# EUROPEAN PATENT APPLICATION

(11) **EP 2 332 557 A1**
(43) Date of publication of application: **15.06.2011**
(21) Application number: 09178387.8
(22) Date of filing: 08.12.2009
(51) Int. Cl.: A61K 35/74, C12N 1/20

(54) **Probiotic lactic acid bacteria**

(71) Applicant: Campina Nederland Holding B.V., 5301 LB Zaltbommel (NL)
(72) Inventor: Snel, Johannes, 6703 GX Wageningen (NL); Kleerebezem, Michiel, 6716 GG Ede (NL); Zwijsen, Renate Maria Louise, 3634 GK Den Dolder (NL); Smit, Bart, 6717 GX Ede (NL); Noordman, Wouter Herman, 6712 HE Ede (NL)
(74) Representative: Ketelaars, Maarten F.J.M.

(57) **Abstract**

The present invention relates to immunomodulating probiotic lactic acid bacteria, to methods wherein the bacteria are used to reduce allergy, and to food products wherein the bacteria may be in incorporated to reduce allergy upon consumption of the product. Preferred probiotic lactic acid bacteria stimulate the Th1 and/or Th3 responses and/or represses Th2 responses *in vivo* as may be determined by the cytokine profiles that are induced in humans upon consumption of the lactic acid bacteria.

## Description

### Field of the invention

The present invention relates to immunomodulating probiotic lactic acid bacteria, to methods wherein the bacteria are used to reduce allergy, and to food products wherein the bacteria may be incorporated to reduce allergy upon consumption of the product.

### Background of the invention

IgE-mediated allergy, also referred to as type I hypersensitivity, is the most important hypersensitivity reaction in the body. It is induced by a strong reaction against certain types of environmental compounds (food molecules, pollen, house dust mite, bee venom, etc) referred to as allergens. Typical type I hypersensitivities associated with IgE antibodies include hay fever, asthma, urticaria, anaphylaxic shock and the like.

Exposure to allergens can result in induction of either immunological tolerance or an active immune response. T-lymphocytes play an important role in directing the immune response. Different subsets of T-lymphocytes can be found in the human body. Allergy is the result of a T-helper type 2-(Th2)-mediated immune response and is characterized by the production of interleukins IL-4, IL-5 and IL-13. IL-4 is the cytokine responsible for the formation of IgE-producing cells. IL-5 stimulates B cell growth and increases immunoglobulin secretion. B cells synthesize IgE under certain conditions. IL-13 contributes to isotype switching in B cells to promote IgE production. Allergy has long been seen as a unbalance between Th2 and Th1-(T-helper type 1) responses, Therefore, it was thought that stimulation of a Th1 response should result in a lowering of a Th2 response. However, recently published evidence suggests that Th2 responses are physiologically normal responses that can exist in the absence of allergy, as well as in the presence of strong Th1 responses. Therefore, a role for T regulatory cells (Treg or Th3) and induction of tolerance has been postulated to control the development of allergy.

Allergens do not directly target T-lymphocytes, but are phagocytized by antigen presenting cells such as dendritic cells that can be found throughout the intestinal tract, the respiratory tract and underneath the skin. Antigen presenting cells process the allergens and express parts of it at their cell surface. These are recognized by T lymphocytes in association with other cell surface markers such as MHC-II and B7-2 molecules.

The response of the T lymphocytes is dependent on the activation state of the antigen presenting cells as well as the cytokines produced by these cells. Non-activated dendritic cells are recognized by a low expression of the surface markers MHC-II and B7-2. These cells produce IL-10, and stimulate regulatory T cells (also called T-suppressor cells or T-helper 3 cells; Th3 cells) leading to tolerance induction. Activated dendritic cells have increased expression of MHC-II and B7-2. These cells may produce high levels of IL-12 and IFN-γ that stimulate formation of T-helper 1 cells (Th1 cells), or produce IL-6 and IL-10 to stimulate T-helper 2 cells (Th2 cells). The latter cell type may lead to allergy induction.

The gastro-intestinal tract contains the largest outer surface area of the human body, and has a dense concentration of non-self molecules, such as food molecules and commensal bacteria, that is in close contact with the locally present mucosal immune system. It is thus not surprising that the intestinal microbiota can play a role in the development of allergy. Indeed different colonization patterns have been found in allergic children compared to normal healthy children. Lactobacilli are an important component of the commensal microflora of humans and are frequently used as probiotics. Therefore Lactobacilli have been investigated for their ability to play a role in the prevention of allergy.

Kalliomaki et al. (Lancet, 2001. 357(9262): p. 1076-9) reported that Lactobacillus GG when given either to bottle fed newborns or to their mothers around the time of birth and during the next few months significantly reduced the chance of these infants of developing a cow's milk allergy. Even at the age of four years, the protective effect could still be observed (Kalliomaki et al., Lancet, 2003. 361(9372): p. 1869-71). However, these studies have focussed on allergy prevention in newborns and do not relate to a reduction of symptoms in patients with an existing allergy. Indeed, in contrast to the promising effects on allergy prevention, no effect of Lactobacillus GG on birch-pollen allergy was observed (Helin et al., Allergy, 2002. 57(3): p. 243-6).

EP1364586 discloses lactic acid bacterial strains belonging to the *Lactobacillus* genus, and in particular *Lactobacillus paracasei* strains that are able to promote the induction of oral tolerance in mice to the cow's milk protein beta-lactoglobulin.

US2005214270 discloses anti-allergic agents comprising as an active ingredient one or more lactic acid bacterial strains of the species Lactobacillus acidophilus or *Lactobacillus fermentum* that suppress the IgE level in an ovalbumin hypersensitized mouse model.

Ishida et al. examined the efficacy of orally administered Lactobacillus acidophilus strain L-92 on perennial allergic rhinitis (J Dairy Sci, 2005. 88(2): p. 527-33.) and on symptoms of Japanese cedar pollen allergy (Biosci Biotechnol Biochem, 2005. 69(9): p. 1652-60). Although significant improvement of clinical symptoms such as nasal and ocular symptom-medication scores was observed, no significant differences in serum antihouse dust mite IgE levels or in Th 1/Th 2 ratio between the 2 groups were seen.

EP1634600 discloses anti-allergic compositions comprising as active ingredient the *Lactobacillus paracasei* KW 3110 strain that induces IL 12 production and reduces IL 4 production in vitro in mouse spleen lymphocytes sensitized with ovalbumin.

WO2008/079009 describes lactic acid bacterial strains with immunomodulating capabilities on Peripheral Blood Mononuclear Cells (PBMCs) *in vitro.*

There is however still a need for further probiotic lactic acid bacterial strains with demonstrated immunomodulating capabilities on humans that may be used in methods for reducing allergy, and that may e.g. be in incorporated into food products to reduce allergy.

### Summary of the invention

In a first aspect, the present invention relates to a lactic acid bacterium that upon *in vivo* consumption by a subject: a) reduces IgE levels in a blood sample of said subject; and b) stimulates T-helper 3 cells (Th3 cells) and/or a Th3 response in *ex vivo* stimulated PBMCs of said subject; after 4 weeks of daily consumption of said lactic acid bacterium, compared to a reference strain *L. plantarum* WCFS 1 consumed in the same form and level by a subject.

In another aspect, the invention is concerned with a lactic acid bacterium that upon *in vivo* consumption by a subject: a) reduces IgE levels in a blood sample of said subject; and b) stimulates T-helper 3 cells (Th3 cells) and/or a Th3 response in *ex vivo* stimulated PBMCs of said subject; after 4 weeks of daily consumption of said lactic acid bacterium.

In an embodiment, said lactic acid bacterium further c) represses T-helper 2 cells (Th2 cells) and/or a Th2 response in *ex vivo* stimulated PBMCs of said subject.

In an embodiment, stimulation of Th3 cells and/or a Th3 response induces IL-10 production.

In another embodiment, repression of Th2 cells and/or a Th2 response repressed induction of IL-5 and/or IL-13.

In an embodiment, the bacterium is consumed by the subject in an amount of 10⁸-10¹² colony-forming units per day.

In an embodiment, the bacterium is consumed in the form of a fermented food product, in particular a fermented dairy product.

In an advantageous embodiment, the bacterium is *Lactobacillus plantarum* strain CBS 125632.

In a second aspect the present invention is concerned with a lactic acid bacterium as defined hereinabove for use as a medicament, in particular for use in the treatment of allergy, preferably an IgE-mediated allergy.

The invention also pertains to a composition comprising a lactic acid bacterium as defined above, wherein the composition is at least one of a food composition, a food supplement, a nutraceutical composition, a pharmaceutical composition and animal feed.

In an embodiment, the composition is a dairy product, preferably a dairy product selected from fermented dairy products, yoghurt, milk, milk-based drinks, buttermilk, yoghurt-based drinks, cheese, butter, ice-cream, a milk-derived dessert, soft curd cheese and fresh cheese.

In a further aspect, the present invention relates to the use of a lactic acid bacterium as defined above for the manufacture of a composition for the treatment of allergy, preferably an IgE-mediated allergy. The composition may be administered in an effective amount, the effective amount comprising between about 1x 10⁸ and about 1x 10¹² colony forming units per day. The composition may be a nutraceutical composition, a pharmaceutical composition, animal feed or a dairy product, preferably a dairy product selected from fermented dairy products, yoghurt, milk, milk-based drinks, buttermilk, yoghurt-based drinks, cheese, butter, ice-cream, a milk-derived dessert, soft curd cheese and fresh cheese.

In a final aspect, the present invention provides for a method for identifying a lactic acid bacterium that upon *in vivo* consumption by a subject: a) reduces IgE levels in a blood sample of said subject; b) stimulates T-helper 3 cells (Th3 cells) and/or a Th3 response in *ex vivo* stimulated PBMCs of said subject; and c) optionally, represses T-helper 2 cells (Th2 cells) and/or a Th2 response in *ex vivo* stimulated PBMCs of said subject; after 4 weeks of daily consumption of said lactic acid bacterium, compared to a reference strain *L. plantarum* WCFS1 consumed in the same form and level by a subject, said method comprising the steps of: allowing a subject to daily consume a lactic acid bacterium during 4 weeks; determining the IgE levels in a blood sample of said subject, IL-10 levels, and, optionally, IL-5 levels and/or IL-13 levels, in *ex vivo* stimulated PBMCs prior to and after daily consumption during 4 weeks of said lactic acid bacterium; and selecting a lactic acid bacterium effecting reduced IgE levels, increased IL-10 levels, and, optionally, reduced IL-5 and/or IL-13 levels after 4 weeks consumption.

### Description of the invention

In a first aspect the present invention relates to lactic acid bacterial strains with immunomodulating capabilities. The lactic acid bacterium has the following immunomodulating capabilities upon *in vivo* consumption by a subject: a) reduces IgE levels in a blood sample of said subject; b) stimulates T-helper 3 cells (Th3 cells) and/or a Th3 response in *ex vivo* stimulated PBMCs of said subject; after 4 weeks of daily consumption of said lactic acid bacterium, compared to a reference strain *L. plantarum* WCFS 1 consumed in the same form and level by a subject.

Also, the present invention relates to a lactic acid bacterium that upon *in vivo* consumption by a subject: a) reduces IgE levels in a blood sample of said subject; and b) stimulates T-helper 3 cells (Th3 cells) and/or a Th3 response in *ex vivo* stimulated PBMCs of said subject; after 4 weeks of daily consumption of said lactic acid bacterium.

The lactic acid bacterial strains may be used as a medicament, preferably they may be used in the treatment of allergy. The term "lactic acid bacteria" is used herein to refer to bacteria, which produce lactic acid as a product of fermentation, including e.g. bacteria of the genus *Lactobacillus, Streptococcus, Lactococcus, Oenococcus, Leuconostoc, Pediococcus, Carnobacterium, Propionibacterium, Enterococcus* and *Bifidobacterium.* The lactic acid bacterial strains of the invention are "probiotics" or "probiotic strains", which term herein refers to strains administered in live, sublethal, or dead form, which have a beneficial effect on the host when ingested (e.g., enterally or by inhalation) by a subject. A "subject" refers herein to a human or non-human animal, in particular a vertebrate.

The allergy that may be treated by the lactic acid bacterial strains of the invention preferably is an IgE-mediated allergy, also referred to as type I hypersensitivity. The treatment may comprise the prevention and/or reduction of environmental allergies such as hay fever, topic dermatitis, bronchial asthma, chronic allergic rhinitis, urticaria, and anaphylactic shock. The allergy to be treated may be caused by a large variety of allergens including: airborne particles (hay fever): (pollen from) grass, weeds, timothy grass, birch trees and mould spores; drugs: penicillin, sulfonamides, salicylates (also found naturally in numerous fruits) and local anaesthetics; foods (food allergy): nuts, peanuts, sesame, seafood, eggs (typically albumen,), peas, beans, soybeans and other legumes, celery and celeriac, soy, milk, wheat (gluten) and corn or maize; insect stings: bee sting venom and wasp sting venom; and animal products (animal allergy): animal hair and dander, cockroach calyx, and dust mite excretion.

The lactic acid bacterial strains that are used as active ingredient in the treatment of allergy have immunomodulating capabilities. Preferably, the lactic acid bacterial strains have one or more of the following immunomodulating capabilities that are advantageous in the treatment of allergy:
a) absence of, or reduced induction of an inflammatory response;
b) stimulation of T-helper 3 cells (Th3 cells) and/or a Th3 response. Th3 cells are also referred to as regulatory T cells or T-suppressor cells and favour induction of tolerance;
c) absence of stimulation and/or repression of T-helper 2 cells (Th2 cells) and/or a Th2 response, which may lead to induction of allergy; and
d) No or moderate stimulation of T-helper 1 cells (Th1 cells) and/or a Th1 response.

In accordance with the invention, a lactic acid bacterium having the capability to reduce induction of an inflammatory response in a subject reduces IgE levels in a blood sample of said subject. IgE levels may be measured by methods which are known to the skilled person. For example, IgE levels may be determined using standard ELISAs, solid-phase displacement radioimmunoassays, double-antibody radioimmunoassays, solid-phase sandwich radioimmunoassays, and nephelometry.

In accordance with the invention, a lactic acid bacterium with the capability to stimulate T-helper 3 cells (Th3 cells) and/or a Th3 response preferably is a lactic acid bacterium that upon *in vivo* consumption by a subject induces IL-10 production. Preferably, the lactic acid bacterium induces significantly more IL-10 than a reference strain does. In an embodiment, the lactic acid bacterium induces at least 110%, 115%, 120%, 125%, 130%, 135%, 140%, 150%, or more, of the amount of IL-10 that is induced under the same conditions by a reference strain *L. plantarum* WCFS 1 or in the absence of any reference strain.. The induction is preferably measured in *ex vivo* stimulated PBMCs of said subject.

The IL-10 levels may be measured by any means known in the art, for example, a commercially available ELISA kit for determination of IL-10 levels, such as from R&D Systems, Minneapolis, M.N., or other manufacturers.

A further preferred lactic acid bacterium with the capability to stimulate T-helper 3 cells (Th3 cells) and/or a Th3 response is a lactic acid bacterium that upon *in vivo* consumption daily during 4 weeks by a subject, preferably an allergic subject, more preferably from a subject with an IgE-mediated allergy, most preferably a subject with a pollen allergy (of which birch pollen are most preferred), significantly induces IL-10 production compared to a reference strain, or compared to the absence of a reference strain. Preferably, said lactic acid bacterium induces at least 10, 20, 30, 40, 50, 60, 70, 80 or 90%, or more, more IL-10 after 4 weeks daily consumption of said lactic acid bacterium than a reference strain *L. plantarum* WCFS 1 does, and/or in the absence of any (reference) strain. The induction is preferably measured in *ex vivo* stimulated PBMCs of said subject. The subject may be an allergic subject, preferably a subject with an IgE-mediated allergy, more preferably a subject with a pollen allergy (of which birch pollen are most preferred).

In an embodiment, said lactic acid bacterium further c) represses T-helper 2 cells (Th2 cells) and/or a Th2 response in *ex vivo* stimulated PBMCs of said subject.

In accordance with the invention, a lactic acid bacterium with the capability to repress and/or at least lacking the capability to stimulate T-helper 2 cells (Th2 cells) and/or a Th2 response (leading to induction of allergy) preferably is a lactic acid bacterium that upon *in vivo* consumption daily during 4 weeks by a subject represses or at least does not stimulate the induction of IL-5 significantly. Preferably, the lactic acid bacterium represses IL-5 to such extent upon *ex vivo* stimulation of PBMCs of a subject about 95%, 90%, 85%, 80%, 75%, 70%, 65%, 60%, 55%, 50% or less of the amount of IL-5 is formed as is formed upon *ex vivo* stimulation of PBMCs under the same conditions of a subject having consumed a reference strain *L. plantarum* WCFS1, or in the absence of any reference strain. Preferably, the subject is a subject with an IgE-mediated allergy, most preferably a subject with a pollen allergy (of which birch pollen are most preferred).

In accordance with the invention, a lactic acid bacterium with the capability to repress and/or at least lacking the capability to stimulate T-helper 2 cells (Th2 cells) and/or a Th2 response (leading to induction of allergy) preferably is a lactic acid bacterium that upon *in vivo* consumption daily during 4 weeks by a subject represses or at least does not stimulate the induction of IL-13 significantly. Preferably, the lactic acid bacterium represses IL-13 to such extent upon *ex vivo* stimulation of PBMCs of a subject about 95%, 90%, 85%, 80%, 75%, 70%, 65%, 60%, 55%, 50% or less of the amount of IL-13 is formed as is formed upon *ex vivo* stimulation of PBMCs under the same conditions of a subject having consumed a reference strain *L. plantarum* WCFS1, or in the absence of any reference strain. Preferably, the subject is a subject with an IgE-mediated allergy, most preferably a subject with a pollen allergy (of which birch pollen are most preferred).

In an embodiment, the lactic acid bacterium of the present invention may further have the capability to stimulate T-helper 1 cells (Th1 cells) and/or a Th1 response, or increase the Th1/Th2 ratio. Such bacterium is preferably a bacterium that upon *in vivo* consumption daily during 4 weeks by a subject increases IFN-γ levels, and/or IFN-γ/IL-5 and/or IFN- y/IL-13 ratios, significantly. Preferably, the lactic acid bacterium induces 100%, 105%, 110%, 115%, 120%, 125% or 130%, of the amount of IFN- y that is induced under the same conditions by a reference strain *L. plantarum* WCFS 1 or in the absence of any reference strain. Preferably, the subject is a subject with an IgE-mediated allergy, most preferably a subject with a pollen allergy (of which birch pollen allergy is most preferred).

The comparison with the reference strain is not absolutely required; it may suffice to measure the levels referred to above prior to and after 4 weeks ingestion of the lactic acid bacterium of the present invention. Similar repressions and stimulations may thus be achieved, albeit in the same subject prior to and after 4 weeks ingestion of hte lactic acid bacterium of the present invention.

As used herein, the term "consumption" equals the term "ingestion".

As used herein, the term "significantly" refers to statistically significant. Significance levels show how likely a result is due to chance. As used herein, the level used to mean something is good enough to be believed, is 0.90. This means that the finding has a 90% chance of being true.

"A reference strain" as used herein, refers to the *Lactobacillus plantarum* WCFS 1 strain. However, the results may also be compared to results obtained in the absence of any (reference) strain, as *Lactobacillus plantarum* WCFS1 was found not to show any significant effect (see group 3).

The levels of cytokines as induced after *in vivo* consumption of the above described lactic acid bacteria of the invention were measured after *ex vivo* stimulation of PBMCs by routine methods known in the art, exemplified in the Examples section. Using such *ex vivo* stimulation method, the capacity of immune cells to respond to antigens can be measured. In the present invention, the response of immune cells to anti-CD3 antibodies and anti-CD28 antibodies and the response of immune cells to Bet v 1 was measured. Anti-CD3 antibodies and anti-CD28 antibodies provides an indication of the general allergy response, whereas Bet v 1 is a well accepted inducer of birch pollen allergy. Thus, the PBMCs of the subjects have been subjected to certain potentially immunomodulating bacteria. *Ex vivo,* in blood drawn from the subjects, it can be measured to what extent the contact with the potentially immunomodulating bacteria has altered its response to allergens.

To measure whether or not cytokine levels have changed after ex vivo stimulation of PBMCs, PBMCs may be isolated from blood samples prior to consumption of the lactic acid bacteria of the invention and after 4 weeks daily consumption of the lactic acid bacteria of the invention. The PBMCs may be stimulated by addition of anti-CD3 and anti-CD28 antibodies, or Bet v 1. Anti-CD3 and anti-CD28 antibodies are indicative of general allergy responses, whereas Bet v 1 is indicative of birch pollen responses. Cytokine levels in samples before and after consumption of the lactic acid bacterium of the invention may be compared for changes in cytokine levels.

The lactic acid bacterial strains of the invention preferably are of the genus *Lactobacillus.* The bacteria should be food-grade, i.e. they should be considered as not harmful, when ingested by a human or animal subj ect. It is understood that non-food grade bacteria, for example pathogenic bacteria, which have been modified so that they are no longer harmful when ingested by a subject, are included within the scope of the invention. The *Lactobacillus* strains may be of the following species: *L. rhamnosus, L. casei, L. paracasei, L. helveticus, L. delbrueckii, L. reuteri, L. brevis, L. crispatus, L. sakei, L. jensenii, L. sanfransiscensis, L. fructivorans, L. kefiri, L. curvatus, L. paraplantarum, L. kefirgranum, L. parakefir, L. fermentum, L. plantarum, L. acidophilus, L. johnsonii, L. gasseri, L. xylosus, L. salivarius* etc. Preferred species of lactic acid bacterial strains are of a species selected from *L. acidophilus, L. plantarum, L. fermentum, L. rhamnosus, L. paracasei, L. acetotolerans, L. reuteri, L. casei,* and L. *paracasei* subsp., more preferred are *L. plantarum* and *L. fermentum,* most preferred is the deposited strain CBS 125632, which was deposited under the Budapest Treaty at the Centraalbureau voor Schimmelcultures on December 2, 2009. A lactic acid bacterial strain of the present invention further should be robust, meaning that it viability upon storage in the final product should be higher than 50% after at least 7 days. Moreover, a preferred lactic acid bacterial strain is able to adhere well to intestinal tract so that it remains in the intestinal tract for longer periods of time and may colonise the intestinal tract.

It is understood that replicates and/or derivatives of the deposited strain or any other strain according to the invention are encompassed by the invention. The term "replicate" refers to the biological material that represents a substantially unmodified copy of the material, such as material produced by growth of micro-organisms, e.g. growth of bacteria in culture media. The term "derivative" refers to material created from the biological material and which is substantially modified to have new properties, for example caused by heritable changes in the genetic material. These changes can either occur spontaneously or be the result of applied chemical and/or physical agents (e.g. mutagenesis agents) and/or by recombinant DNA techniques as known in the art. When referring to a strain "derived" from another strain, it is understood that both "replicates" of that strain, as well as "derivatives" of the strain are encompassed, as long as the derived strain still retains the immunomodulating capabilities of the strain from which it was derived, and therefore can be used in the treatment of allergy.

In another aspect the invention relates to the use of a lactic acid bacterial strain as described above for the preparation (manufacture) of a composition for the treatment allergy as defined herein above. The composition that is manufactured using one or more strain(s) according to the invention may be any type of composition, which is suitable for consumption by, or administration to a subject, preferably a human subject suffering from an allergy as defined above. The composition may be a food, a food supplement composition, a nutraceutical or a pharmaceutical composition. Depending on the type of composition and its preferred administration method, the components and texture of the composition may vary. A food or food/nutritive composition comprises besides the bacterial strain(s) of the invention also a suitable food base. A food or food composition is herein understood to include solids (for example powders, such as baby formula powders), semi-solids and/or liquids (e.g. a drink or beverage) for human or animal consumption. A food or food/nutritive composition may be a dairy product, such as fermented dairy products, yoghurt, milk or milk-based drinks, buttermilk, yoghurt-based drinks, cheese and butter, ice-cream, desserts (e.g. milk-derived desserts like custards), soft curd cheese, fresh cheese (e.g. cottage cheese), etc. Such foods or food compositions may be prepared in a manner known per se, e.g. by adding the strain(s) of the invention to a suitable food or food base, in a suitable amount (see e.g. WO 01/82711). In a further embodiment, the strain(s) are used in or for the preparation of a food or food/nutrient composition, e.g. by fermentation. Examples of such strains include probiotic lactic acid producing bacteria of the invention. In doing so, the strain(s) of the invention may be used in a manner known per se for the preparation of such fermented foods or food/nutrition compositions, e.g. in a manner known per se for the preparation of fermented dairy products using lactic acid producing bacteria. In such methods, the strain(s) of the invention may be used in addition to the micro-organism usually used, and/or may replace one or more or part of the micro-organism usually used. For example, in the preparation of fermented dairy products such as yoghurt or yoghurt-based drinks, a live food grade lactic acid producing bacterium of the invention may be added to or used as part of a starter culture or may be suitably added during or after such a fermentation. Also flavourings, anti-oxidants, vitamins, minerals, colouring agents, etc may be present. Although preferably living cells are used, dead or non-viable cells may also be used in some compositions.

A preferred lactic acid bacterial strain of the invention is a strain that is stable in a dairy product, particularly in a fermented dairy product. Stability of the strain is herein understood as survival of viable bacteria after prolonged storage in a (fermented) dairy product. Preferably a lactic acid bacterial strain of the invention is stable in a (fermented) dairy product under refrigeration conditions (<7°C) for at least 1, 2, 3, or 4 weeks whereby it is understood that the survival log of the bacterial strain (i.e. cfu after storage / cfu at start) is at least 0.5.

Apart from an effective amount of one or more of the lactic acid bacterial strains of the invention, a food supplement may comprise one or more carriers, stabilizers, prebiotics and the like. Preferably, the composition is in powder form, for enteral (preferably oral) administration, although nasal administration or inhalation may also be suitable. When using living cells of the strain(s), the cells may be present in an encapsulated form in order to be protected against the stomach juices which means that in this case the cells do not need to be resistant to digestive juice. The composition may e.g. be in the form of a powder packed in a sachet which can be dissolved or dispersed in water, fruit juice, milk or another beverage. The dose of cells is preferably at least at least 1x10⁶ cfu, preferably between about 1x10⁶ - 1x10¹² cfu (colony forming units) per day, more preferably between about 1x10⁷ - 1x10¹² cfu/day, more preferably about 1x10⁸ ― 1x10¹², even more preferably about 1x10⁸ ― 5x10¹⁰ cfu/day, most preferably between 1x10⁹-2x10¹⁰ cfu/day. The effective dose may be provided as a single dosage or may be subdivided into several smaller dosages and administered for example in two, three or more portions per day.

Apart from one or more of the lactic acid bacterial strains of the invention, a nutritional composition preferably comprises carbohydrates and/or proteins and/or lipids suitable for human and/or animal consumption. The compositions may or may not contain other bioactive ingredients, such as other (probiotic) strains, and prebiotics, which support the probiotic strains. When using living cells of the strain(s), the cells may be present in an encapsulated form in order to be protected against the stomach juice. The dose of living cells per strain is preferably at least 1x10⁶ cfu, preferably between about 1x10⁶ - 1x10¹² cfu (colony forming units) per day, more preferably between about 1x10⁷ - 1x10¹² cfu/day, more preferably about 1x10⁸ ― 1x10¹², even more preferably about 1x10⁸ ― 5x10¹⁰ cfu/day, most preferably between 1x10⁹-2x10¹⁰ cfu/day. The nutritional composition may replace the normal food/drink intake of a subject, or may be consumed in addition thereto.

It is understood that when dead or non-viable cells dosages equivalent to the above cfu's are used. These equivalent dosages may be e.g. be determined using optical density (e.g. OD₆₀₀) or by protein or DNA quantification.

One or more of the lactic acid bacterial strains of the invention in a suitable dosage may also be used to make a nutraceutical or pharmaceutical composition for treatment, therapy or prophylaxis of allergy. Nutraceutical/pharmaceutical compositions will usually be used for enteral, for example oral application. Nutraceutical/pharmaceutical compositions will usually comprise a pharmaceutical carrier in addition to the strain(s) of the invention. The preferred form depends on the intended mode of administration and (therapeutic) application. The pharmaceutical carrier can be any compatible, non-toxic substance suitable to deliver the strains(s) to the desired body cavity, e.g. the intestine of a subject. E.g. sterile water, or inert solids may be used as the carrier usually complemented with pharmaceutically acceptable adjuvants, buffering agents, dispersing agents, and the like. Nutraceutical/pharmaceutical compositions may further comprise additional biologically or pharmaceutically active ingredients.

In yet a further aspect, the invention pertains to a method for preparing a composition for the treatment of allergy, comprising the steps of: a) growing at least one lactic acid bacterial strain as defined herein in a suitable liquid or solid medium; b) optionally isolating the strain from the medium, for example by centrifugation and/or filtration and performing down stream processing as known in the art, for example lyophilisation, spray drying and/or freezing; and, c) formulating the strain into a form suitable for administration to a subject. The strains of the invention may be grown on artificial media or on natural media, such as (low fat) milk, yoghurt, and the like. It may then be used directly to make a composition according the invention, or the bacteria may be concentrated or isolated by centrifugation and/or filtration from the medium and then formulated into suitable compositions.

Finally, the present invention provides for a method for identifying a lactic acid bacterium that upon *in vivo* consumption by a subject: a) reduces IgE levels in a blood sample of said subject; b) stimulates T-helper 3 cells (Th3 cells) and/or a Th3 response in *ex vivo* stimulated PBMCs of said subject; and c) optionally, represses T-helper 2 cells (Th2 cells) and/or a Th2 response in *ex vivo* stimulated PBMCs of said subject; after 4 weeks of daily consumption of said lactic acid bacterium, compared to a reference strain *L. plantarum* WCFS 1 consumed in the same form and level by a subject, said method comprising the steps of: allowing a subject to daily consume a lactic acid bacterium during 4 weeks; determining the IgE levels in a blood sample of said subject, IL-10 levels, and, optionally, IL-5 levels and/or IL-13 levels, in *ex vivo* stimulated PBMCs prior to and after daily consumption during 4 weeks of said lactic acid bacterium; and selecting a lactic acid bacterium effecting reduced IgE levels, increased IL-10 levels, and, optionally, reduced IL-5 and/or IL-13 levels after 4 weeks consumption.

Further embodiments of this method are set forth above with reference to the lactic acid bacterium of the invention. For example, IFN-γ levels may be measured in *ex vivo* stimulated PBMCs prior to and after daily consumption during 4 weeks of said lactic acid bacterium to additionally determine the Th1 response as indicated above.

In this document and in its claims, the verb "to comprise" and its conjugations is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

It will be clear that the above description and drawings are included to illustrate some embodiments of the invention, and not to limit the scope of protection. Starting from this disclosure, many more embodiments will be evident to a skilled person which are within the scope of protection and the essence of this invention and which are obvious combinations of prior art techniques and the disclosure of this patent.

### Examples

### Example 1: Preparation of yogurts

Yogurt was prepared comprising no probiotic lactic acid bacterium (placebo yogurt), a yogurt comprising *Lactobacillus casei* (positive control), or a yogurt comprising one of 4 selected strains: *L. plantarum* WCFS1, *L. plantarum* B3400, *L. plantarum* B2877, and *L. plantarum* CBS 125632). The 4 selected strains had previously demonstrated immunomodulating capabilities *in vitro:* upon co-incubation *in vitro* with human PBMCs they induced IL-12 and/or IFN-γ, and/or induced IL-10 and/or IL-1β, and/or repressed or at least did not stimulate IL-13.

Yoghurts were prepared by pasteurising skimmed milk (protein 2.8-3.7%, sugar 4-5% and fat <0.1%). After cooling down to fermentation temperature of said 37°, a yogurt starter was added (Placebo). In addition, the following strains are inoculated *Lactobacillus casei* (positive control), *L. plantarum* WCFS1, *L. plantarum* B3400, L. *plantarum* B2877, and *L. plantarum* CBS 125632. The consortia of lactic acid bacteria fermented the milk for 10-14 hours to a final pH of 4.0-4.4. After this fermentation 5-15% lemon-lime flavoured syrup was mixed with the fermentate, and the mix was pumped, sheared, cooled, and packed in portions of 100 mL, each containing ca. 10¹⁰ CFU per 100 ml.

### Example 2: Double-blind placebo-controlled study on clinical effects of selected strains

### Inclusion criteria:

● Anamnestic hay fever complaints
● Positive RAST or intracutaneous skin test for birch pollen
● Age: 18-50 years; no restrictions with respect to gender/ethnicity

### Exclusion criteria:

● Use of probiotics during the last month prior to the study
● Sensitivity for pets if present in direct habitat
● Use of medication influencing the immune system
● Use of antibiotics over the last 2 months prior to the study
● Lactose intolerance
● Pregnancy
● Blood picture indicative of infection

### Set-up of the clinical study

62 subjects with confirmed allergy for birch pollen were included. Prior to the study, the subjects were randomized based on gender, age, and last documented severity of hay fever. 10 subjects were randomly assigned to the group receiving placebo yogurts, 10 subjects were assigned to the group receiving yogurt comprising *Lactobacillus casei* (positive control), 9 subjects were assigned to the group receiving yogurt comprising *L. plantarum* WCFS1, 10 subjects were assigned to the group receiving yogurt comprising *L. plantarum* B3400, 11 subjects were assigned to the group receiving yogurt comprising *L. plantarum* B2877, and 10 subjects were assigned to the group receiving yogurt comprising *L. plantarum* CBS 125632. The study was conducted out of the pollen season to prevent changes in the blood picture of subjects due to attacks of hay fever.

Prior to the study, 50 ml blood was drawn from the subjects through venapunction in the arm. The intervention started the next day. The subjects daily consumed 100 ml yogurt comprising 10¹⁰ colony-forming units (cfu) during 4 weeks. They were requested to refrain from consuming other probiotic products, regular yogurt or buttermilk during the intervention period. 4 weeks after start of the intervention study, again 50 ml blood was drawn from the subjects through venapunction in the arm.

The blood samples were subjected to various direct measurements (sedimentation, CRP, leucocyte counts, immunoglobulines). In addition, PBMCs were cultured *ex vivo* and stimulated with relevant allergens. Cytokine (IL-5, IL-10, IL-13) and surface marker (CD3, CD4, CD8, CD19) levels were determined. The individual parameters at the start and the end of the study were compared.

### Direct measurements

Birch-pollen specific IgE levels were measured using ImmunoCAP (Phadia AB, Uppsala, Sweden).

### Ex vivo stimulation of PBMCs of subjects

To study the capacity of immune cells to react to antigens following ingestion of the lactic acid bacteria of the invention, *ex vivo* stimulation of PBMCs isolated from the subjects was performed. PBMCs were isolated from the blood samples drawn from the subjects by centrifugation over Ficoll-Paque. After washing, the cells were resuspended in RPMI 1640 medium containing 10% heat-inactivated fecal calf serum, 2 mM glutamine, and the antibiotics penicillin and streptomycin. Blood cells were stimulated by addition of anti-CD3 and anti-CD28 antibodies, or Bet v 1. Anti-CD3 and anti-CD28 antibodies are indicative of general allergy responses, whereas Bet v 1 is indicative of birch pollen responses. Cytokines were determined by flow cytometric analysis, using labelled antibodies against the cytokines. Cytokine production by PBMC was analyzed in supernatants of cells cultured for 4 (anti-CD3/CD28 stimulated) or 7 days (Bet v 1 stimulated). The production of the innate and adaptive cytokines was detected using Cytometric Bead Array (CBA, BD Biosciences, San Diego, CA). All buffers used in this protocol were obtained from the BD CBA Soluble Protein Master Buffer Kit (BD Pharmingen) and the procedure was performed according to the manufacturer's protocol. In this respect, reference is made to Jeurink et al. (2008, Cryobiology 57:91-103).

### Measurement of cytokines

The following cytokines were measured:
● IL-5
● IL-13
● IL-10
● Interferon-γ

### Results of the clinical study

The control group, receiving placebo yogurt (group 1), did not show any changes in antibody or surface marker levels in time. Also, no change in cytokine profile of PBMC could be observed after α-CD3/CD28 stimulation or after Bet v 1-stimulation.

The positive control group, receiving yogurt comprising *Lactobacillus casei* (group 2) demonstrated a significant reduction of IgE (p<0.05). Also, the present inventors observed a decrease in the percentage of necrotic cells from the median value 12.6% to 8.7% (p<0.10), and a reduction in CD16+/CD56+ NK cells from 11.4% (median visit 1) to 8.5% (visit 2).
The group receiving yogurt comprising *L. plantarum* WCFS1 (group 3) demonstrated a significant reduction (p<0.10) in IgE levels.
The group receiving yogurt comprising *L. plantarum* B3400 (group 4) demonstrated a significant reduction (p<0.10) in IgE levels. Also, IL-13 levels increased after 7 days Bet v 1 stimulation (p<0.05).
The group receiving yogurt comprising *L. plantarum* B2877 (group 5) had an increased IL-10/IL-5 ratio after a aCD3/aCD28 stimulation (p<0.05). Also, a slight increase in CD14+ monocytes from 18.8% (median visit 1) to 19.3% (median visit 2) (p<0.05) was observed.
The group receiving yogurt comprising *L. plantarum* CBS 125632 (group 6) showed a significant decrease in IL-5 levels (p<0.10) 4 days after a aCD3/aCD28 stimulation. Also, IL-13 levels were significantly (p<0.10) decreased, and IL-10 levels were significantly increased 4 days after a aCD3/aCD28 stimulation. CD19+ B-lymphocyte levels were significantly reduced (p<0.10). IgE levels were significantly reduced (p<0.10). Other markers of interest in group 6 were the following: IL-5 reduction at 7 days after Bet v 1 stimulation (p=0.10), reduction of percentage apoptotic PBMC cells: median from 29.6% (visit 1) to 26.4% (visit 2)(p<0.05), increase in number of living PBMC cells from 60.5% (median visit 1) to 66.5% (median visit 2)(p<0.05), significant increase in IFNγ /IL-13 ratio (p<0.10) in time after a aCD3/aCD28 stimulation, and a significant increase in IFNγ/IL-5 ratio (p<0.05) after a aCD3/aCD28 stimulation.

In summary, strain CBS 125632 reduced IgE antibodies in serum, stimulated Th3 responses (as determined by IL10), and reduced Th2 responses (as determined by IL5 and IL13), and thus improved immune markers related to IgE-mediated allergy.

## Claims

1. A lactic acid bacterium that upon *in vivo* consumption by a subject:
a) reduces IgE levels in a blood sample of said subject;
b) stimulates T-helper 3 cells (Th3 cells) and/or a Th3 response in *ex vivo* stimulated PBMCs of said subject;
after 4 weeks of daily consumption of said lactic acid bacterium, compared to a reference strain *L. plantarum* WCFS 1 consumed in the same form and level by a subject.

2. A lactic acid bacterium that upon *in vivo* consumption by a subject:
a) reduces IgE levels in a blood sample of said subject;
b) stimulates T-helper 3 cells (Th3 cells) and/or a Th3 response in *ex vivo* stimulated PBMCs of said subject;
after 4 weeks of daily consumption of said lactic acid bacterium.

3. A lactic acid bacterium according to claim 1 or 2, which further c) represses T-helper 2 cells (Th2 cells) and/or a Th2 response in *ex vivo* stimulated PBMCs of said subj ect.

4. A lactic acid bacterium according to any of the preceding claims, wherein stimulation of Th3 cells and/or a Th3 response induces IL-10 production.

5. A lactic acid bacterium according to claim 3 or 4, wherein repression of Th2 cells and/or a Th2 response repressed induction of IL-5 and/or IL-13.

6. A lactic acid bacterium according to any of the preceding claims, wherein the bacterium is consumed by a subject, preferably a human, in an amount of 10⁸-10¹² colony-forming units per day.

7. A lactic acid bacterium according to any of the preceding claims, which is consumed in the form of a fermented food product, in particular a fermented dairy product.

8. *Lactobacillus plantarum* strain CBS 125632.

9. A lactic acid bacterium according to any one of claims 1 - 8, for use as a medicament.

10. A lactic acid bacterium according to claim 9, for use in the treatment of allergy, preferably an IgE-mediated allergy.

11. A composition comprising a lactic acid bacterium as defined in any one of claims 1 - 8, wherein the composition is at least one of a food composition, a food supplement, a nutraceutical composition, a pharmaceutical composition, or an animal feed composition.

12. A composition according to claim 11, wherein the composition is a dairy product, preferably a dairy product selected from fermented dairy products, yoghurt, milk, milk-based drinks, buttermilk, yoghurt-based drinks, cheese, butter, ice-cream, a milk-derived dessert, soft curd cheese and fresh cheese.

13. Use of a lactic acid bacterium as defined in any one of claims 1 - 8, for the manufacture of a composition for the treatment of allergy, preferably an IgE-mediated allergy.

14. A use according to claim 13, wherein the composition is administered in an effective amount, the effective amount comprising between about 1x10⁶ - 1x10¹² colony forming units per day.

15. A use according to claims 13 or 14, wherein the composition is a nutraceutical composition, a pharmaceutical composition or a dairy product, preferably a dairy product selected from fermented dairy products, yoghurt, milk, milk-based drinks, buttermilk, yoghurt-based drinks, cheese, butter, ice-cream, a milk-derived dessert, soft curd cheese and fresh cheese.

16. A method for identifying a lactic acid bacterium that upon *in vivo* consumption by a subj ect:
a) reduces IgE levels in a blood sample of said subject;
b) stimulates T-helper 3 cells (Th3 cells) and/or a Th3 response in *ex vivo* stimulated PBMCs of said subject; and
c) optionally, represses T-helper 2 cells (Th2 cells) and/or a Th2 response in *ex vivo* stimulated PBMCs of said subject;
after 4 weeks of daily consumption of said lactic acid bacterium, compared to a reference strain *L. plantarum* WCFS 1 consumed in the same form and level by a subject, said method comprising the steps of:
allowing a subject to daily consume a lactic acid bacterium during 4 weeks;
determining the IgE levels in a blood sample of said subject, IL-10 levels, and, optionally, IL-5 levels and/or IL-13 levels, in *ex vivo* stimulated PBMCs prior to and after daily consumption during 4 weeks of said lactic acid bacterium; and
selecting a lactic acid bacterium effecting reduced IgE levels, increased IL-10 levels, and, optionally, reduced IL-5 and/or IL-13 levels after 4 weeks consumption.
